# EUROPEAN PATENT APPLICATION

(11) **EP 4 159 103 A1**
(43) Date of publication of application: **05.04.2023**
(21) Application number: 21817530.5
(22) Date of filing: 27.05.2021
(51) Int. Cl.: A61B 1/00

(54) **COMMUNICATION METHOD FOR CAPSULE SYSTEM**

(30) Priority: 01.06.2020 CN 202010486167
(71) Applicant: Ankon Medical Technologies (Shanghai) Co., Ltd, Shanghai 200131 (CN)
(72) Inventor: DUAN, Xiaodong, Shanghai 201206 (CN); DU, Jianming, Shanghai 201206 (CN)
(74) Representative: Lavoix
(86) International application number: PCT/CN2021/096443
(87) International publication number: WO 2021/244397

(57) **Abstract**

A communication method for a capsule system, comprising: a capsule periodically transmits a capsule characteristics identifier; a configurator receives the capsule characteristics identifier, and transmits the capsule characteristics identifier and a configurator characteristics identifier of the configurator; the capsule checks whether the received capsule characteristics identifier is consistent with the capsule characteristics identifier in local, and when it is consistent, the capsule saves the configurator characteristics identifier and transmits the configurator characteristics identifier; the configurator receives and checks whether the configurator characteristics identifier is consistent with the configurator characteristics identifier in local, and when it is consistent, the configurator locks the capsule to complete pairing.

## Description

### CROSS-REFERENCE TO RELATED APPLICATION

The application claims priority from Chinese Patent Application No. 202010486167.9, filed June 1, 2020, entitled "Communication method for capsule system", all of which are incorporated herein by reference in their entirety.

### TECHNICAL FIELD

The present invention relates to a swallowable device technology, and more particularly to a communication method for a capsule system.

### BACKGROUND

Currently, capsule apparatuses are widely used in clinical practice. Among them, capsule endoscope has achieved great outcomes in examination of subjects' gastrointestinal tract and has shown significant advantages over traditional endoscopy technique in examination of subjects' small bowel, allowing examination of areas of the gastrointestinal tract that cannot be examined in traditional endoscopy. Another example is that a vibrating capsule can effectively relieve colon spasm, promote colon movement, treat constipation and facilitate bowel movement by vibrating on colon wall of the subject, thus achieving the effect of maintaining beauty and health.

The inside of a capsule can accommodate a processor chip of a wireless communication circuit and its auxiliary circuits, and can enable transmission of commands and configuration parameters via wireless communication technology. Therefore, the capsule can communicate with a configurator even while inside the subject and change its operating mode as often as needed.

However, if a plurality of capsules or a plurality of configurators appear in an area at the same time, wireless communication signals are confused, resulting in loss of control of the capsules.

Therefore, it is needed to design a communication method for capsule systems that can lock paired capsules and configurators.

### SUMMARY

To solve one of the above problems, the present invention discloses a communication method for a capsule system, the communication method comprising: a capsule periodically transmits a capsule characteristic identifier; a configurator receives the capsule characteristic identifier, and transmits the capsule characteristic identifier and a configurator characteristic identifier of the configurator; the capsule receives the capsule characteristic identifier and checks whether the received capsule characteristic identifier is consistent with the capsule characteristic identifier in local, and when the received capsule characteristic identifier is consistent with the capsule characteristic identifier in local, the capsule saves the configurator characteristic identifier and transmits the configurator characteristic identifier; the configurator receives the configurator characteristic identifier and checks whether the received configurator characteristic identifier is consistent with the configurator characteristic identifier in local, and when the received configurator characteristic identifier is consistent with the configurator characteristic identifier in local, the configurator locks the capsule to complete pairing.

In one embodiment, the communication method specifically comprises: the capsule periodically transmits a capsule communication packet that comprises the capsule characteristic identifier and a product code; the configurator receives the capsule communication packet, extracts the capsule characteristic identifier and the product code, adds them together with the configurator characteristic identifier to a pairing command, and transmits the pairing command; the capsule receives the pairing command and checks whether the capsule characteristic identifier and the product code in the pairing command are consistent with the capsule characteristic identifier and the product code in local; and when the capsule characteristic identifier and the product code in the pairing command are consistent with the capsule characteristic identifier and the product code in local, the capsule saves the configurator characteristic identifier in the pairing command and adds the configurator characteristic identifier to the capsule communication packet and transmits the capsule communication packet; the configurator receives the capsule communication packet and checks whether the configurator characteristic identifier in the capsule communication packet is consistent with the configurator characteristic identifier in local, and when the configurator characteristic identifier in the capsule communication packet is consistent with the configurator characteristic identifier in local, the configurator locks the capsule to complete pairing.

In one embodiment, the capsule communication packet comprises a command characteristic identifier, capsule characteristic identifier bits, capsule state information, and configurator characteristic identifier bits, with the capsule characteristic identifier bits optionally filled in with the capsule characteristic identifier and the configurator characteristic identifier bits optionally filled in with the configurator characteristic identifier. When the command characteristic identifier is a product code identifier, the capsule communication packet is product identification information communication data; when the command characteristic identifier is a capsule state identifier, the capsule communication packet is capsule state communication data.

In one embodiment, in the step "the capsule receives the pairing command and checks whether the capsule characteristic identifier and the product code in the pairing command are consistent with the capsule characteristic identifier and the product code in local", when the capsule characteristic identifier and the product code in the pairing command are consistent with the capsule characteristic identifier and the product code in local, the capsule is controlled to enter a data communication state, and the capsule periodically transmits the product identification information communication data and the capsule state communication data in turn, and both of the product identification information communication data and the capsule state communication data comprise the capsule state information. After the step "the configurator locks the capsule" comprises: the configurator saves the product identification information communication data and continuously acquires the capsule state information from the capsule state communication data.

In one embodiment, the communication method specifically comprises: the capsule periodically transmits a capsule broadcast packet that comprises the capsule characteristic identifier; the configurator finds the capsule, extracts the capsule characteristic identifier and adds it to a configurator pairing packet along with the configurator characteristic identifier and a data communication channel, and transmits the configurator pairing packet; the capsule receives the configurator pairing packet and checks whether the capsule characteristic identifier in the configurator pairing packet is consistent with the capsule characteristic identifier in local; and when the capsule characteristic identifier in the configurator pairing packet is consistent with the capsule characteristic identifier in local, the capsule saves the configurator characteristic identifier locally and adds the configurator characteristic identifier to the capsule broadcast packet and transmits it, and transmits a capsule information packet through the data communication channel in the configurator pairing packet; the configurator receives the capsule broadcast packet and checks whether the configurator characteristic identifier in the capsule broadcast packet is consistent with the configurator characteristic identifier in local, and when the configurator characteristic identifier in the capsule broadcast packet is consistent with the configurator characteristic identifier in local, the configurator locks the capsule to complete pairing.

In one embodiment, before the step "the configurator locks the capsule to complete the pairing" comprises: a first antenna of the configurator is on a monitoring channel and a second antenna of the configurator is on the data communication channel; after the step "the configurator locks the capsule" comprises: the first antenna of the configurator and the second antenna of the configurator are both on the data communication channel, and receive the capsule information packet together to continuously obtain the capsule state information.

In one embodiment, the capsule broadcast packet comprises the command characteristic identifier, the capsule characteristic identifier bits, a capsule power-on time, and the configurator characteristic identifier bits; the configurator pairing packet comprises the command characteristic identifier, the capsule characteristic identifier bits, the data communication channel, and the configurator characteristic identifier bits; the capsule information packet comprises the command characteristic identifier, the capsule characteristic identifier bits, information data, and the configurator characteristic identifier bits; the capsule characteristic identifier bits are optionally filled in with the capsule characteristic identifier and the configurator characteristic identifier bits are optionally filled in with the configurator characteristic identifier.

In one embodiment, after the step "the configurator locks the capsule" comprises: a user mobile terminal transmits the product code of the currently paired capsule to the configurator, the configurator transmits an unlock command to the currently paired capsule, the capsule receives the unlock command and transmits an unlock confirmation command to the configurator, the configurator receives the unlock confirmation command and unpairs with the capsule; or, the configurator receives a mechanical key signal, the configurator transmits an unlock command to the currently paired capsule, the capsule receives the unlock command and transmits an unlock confirmation command to the configurator, the configurator receives the unlock confirmation command and unpairs with the capsule; or, the configurator monitors duration of the capsule power-on time, and the capsule is automatically unpaired with the configurator when the duration of the capsule power-on time reaches a threshold T.

In one embodiment, after the step "the configurator locks the capsule" comprises: a target configurator obtains the product code of a target capsule; unpairing the current paired capsule of the target configurator; when the target capsule is in an unpaired state, pairing the target configurator with the target capsule; when the target capsule is in a paired state, the target configurator transmits a forced unpair command to the target capsule to unpair the target capsule with the current configurator, and then pairing the target configurator with the target capsule.

In one embodiment, the step "a target configurator obtains the product code of a target capsule" comprises: the user mobile terminal obtains the product code of the target configuration capsule and transmits it to the configurator; or, the user mobile terminal receives the capsule searched by the target configurator and receives a selection information to select the target configuration capsule, and then transmits the product code of the selected target configuration capsule to the configurator.

In contrast to the prior art, the capsule pairs the received capsule characteristic identifier with the capsule characteristic identifier in local, and if it is consistent, the capsule characteristic identifier transmitted by the capsule is received by a configurator, and the capsule and the configurator go to a first stage of verification; then, the configurator pairs the received configurator characteristic identifier with the configurator characteristic identifier in local, and if it is consistent, the configurator characteristic identifier transmitted by the configurator is received by the corresponding capsule, and the capsule and the configurator go to a second stage of verification. In addition, during the two verifications, the capsule saves the configurator characteristic identifier of the corresponding configurator, and the configurator saves the capsule characteristic identifier of the corresponding capsule. Therefore, the capsule and the configurator can be paired with each other, and subsequent communication between the two can be one-to-one without interference from other capsules or configurators.

### BRIEF DESCRIPTION OF THE DRAWINGS

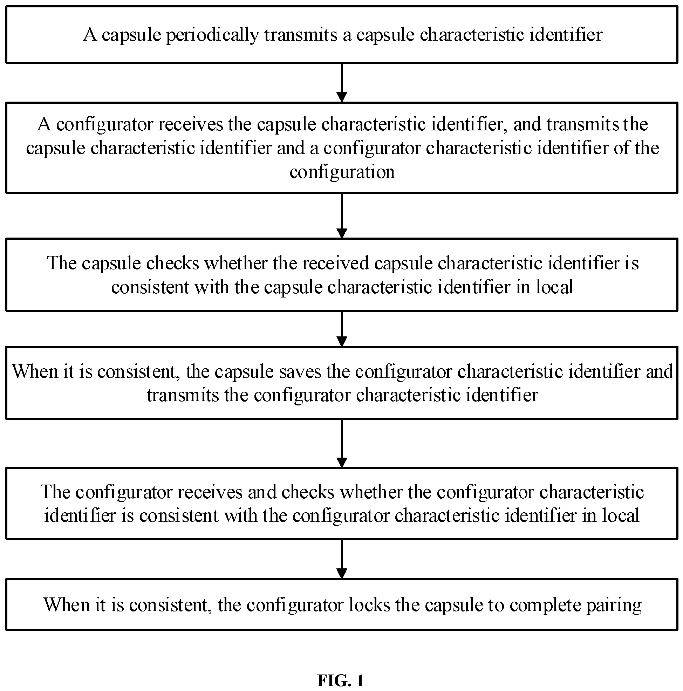

FIG. 1 shows a process flow diagram of a communication method for a capsule system in accordance with the present invention.

### DETAILED DESCRIPTION

In order to enable those in the art to better understand technical solutions in the present invention, the technical solutions in the embodiments of the present invention will be clearly and completely described below in conjunction with accompanying drawings in the embodiments of the present invention. It is clear that the embodiments described are only a part of the embodiments of the present invention, and the present invention is capable of other embodiments or of being practiced or carried out in various ways. Based on the embodiments in the present invention, all other embodiments obtained by a person of ordinary skill in the art without making creative labor shall fall within the scope of protection of the present invention.

Referring to FIG.1, the present invention discloses a communication method for a capsule system, the communication method comprising:
a capsule periodically transmits a capsule characteristic identifier;
a configurator receives the capsule characteristic identifier, and transmits the capsule characteristic identifier and a configurator characteristic identifier of the configurator;
the capsule receives the capsule characteristic identifier and checks whether the received capsule characteristic identifier is consistent with the capsule characteristic identifier in local, and when the received capsule characteristic identifier is consistent with the capsule characteristic identifier in local, the capsule saves the configurator characteristic identifier and transmits the configurator characteristic identifier;
the configurator receives the configurator characteristic identifier and checks whether the received configurator characteristic identifier is consistent with the configurator characteristic identifier in local, and when the received configurator characteristic identifier is consistent with the configurator characteristic identifier in local, the configurator locks the capsule to complete pairing.

Therefore, in the present invention, the capsule pairs the received capsule characteristic identifier with the capsule characteristic identifier in local, and if the received capsule characteristic identifier is consistent with the capsule characteristic identifier in local, the capsule characteristic identifier transmitted by the capsule has been received by a configurator, and the capsule and the configurator go to a first stage of verification; then, the configurator pairs the received configurator characteristic identifier with the configurator characteristic identifier in local, and the received configurator characteristic identifier is consistent with the configurator characteristic identifier in local, the configurator characteristic identifier transmitted by the configurator has been received by the corresponding capsule, and the capsule and the configurator go to a second stage of verification. In addition, during the two verifications, the capsule saves the configurator characteristic identifier of the corresponding configurator, and the configurator saves the capsule characteristic identifier of the corresponding capsule. Therefore, the capsule and the configurator can be paired with each other, and subsequent communication between the two can be one-to-one without interference from other capsules or configurators.

The invention may have a variety of embodiments due to different types of data transmitted by the capsule and the configurator. Specifically, the present invention provides two specific embodiments, which are described in detail below.

In the first embodiment of the present invention, the capsule transmits a capsule communication packet. Specifically, the communication method specifically comprises:
the capsule periodically transmits a capsule communication packet that comprises the capsule characteristic identifier and a product code;
the configurator receives the capsule communication packet, extracts the capsule characteristic identifier and the product code, adds them together with the configurator characteristic identifier to a pairing command, and transmits the pairing command;
the capsule receives the pairing command and checks whether the capsule characteristic identifier and the product code in the pairing command are consistent with the capsule characteristic identifier and the product code in local;
and when the capsule characteristic identifier and the product code in the pairing command are consistent with the capsule characteristic identifier and the product code in local, the capsule saves the configurator characteristic identifier in the pairing command and adds the configurator characteristic identifier to the capsule communication packet and transmits the capsule communication packet;
the configurator receives the capsule communication packet and checks whether the configurator characteristic identifier in the capsule communication packet is consistent with the configurator characteristic identifier in local, and when the configurator characteristic identifier in the capsule communication packet is consistent with the configurator characteristic identifier in local, the configurator locks the capsule to complete pairing.

At different stages, the capsule communication packets may differ in contents. Specifically, the capsule communication packet is composed of 10 bytes, comprising 1 byte of a command characteristic identifier, 2 bytes of capsule characteristic identifier bits, 5 bytes of capsule state information, and 2 bytes of configurator characteristic identifier bits. Wherein, the command characteristic identifier may be a product code identifier, or a capsule state identifier; the capsule state information may be the product code, an operating state, a command confirmation, test data, etc. The capsule characteristic identifier bits can be optionally filled in with the capsule characteristic identifier, and the configurator characteristic identifier bits can be optionally filled in with the configurator characteristic identifier.

In the initial pairing process, the command characteristic identifier is the product code identifier, the capsule communication packet is actually product identification information communication data, and the capsule characteristic identifier bits are filled with the capsule characteristic identifier, and the configurator characteristic identifier bits are empty, without the configurator characteristic identifier. In the process of pairing confirmation, the command characteristic identifier is the product code identifier, the capsule communication packet is actually also the product identification information communication data, and the capsule characteristic identifier bits are filled with the capsule characteristic identifier, and the configurator characteristic identifier bits are filled with the configurator characteristic identifier. After the capsule and the configurator are interlocked and start communicating properly, the command characteristic identifier is the capsule state identifier and the capsule communication packet is actually capsule state communication data.

Therefore, in the first embodiment of the present invention, the communication method actually comprises:
the capsule periodically transmits the product identification information communication data that comprises the capsule characteristic identifier and the product code; in the initial pairing process, the capsule characteristic identifier bits are filled with the capsule characteristic identifier and the configurator characteristic identifier bits are empty. After the capsule is powered up, the capsule is in an information broadcast mode, and transmits the product identification information communication data at fixed intervals (e.g., 2 seconds + N milliseconds) as a handshake command.

The configurator receives the product identification information communication data, extracts the capsule characteristic identifier and the product code, adds them together with the configurator characteristic identifier to the pairing command, and transmits the pairing command; the configurator identifies the product code and thereby confirms that there is a vacant capsule searching for a paired configurator; the configurator does not transmit the product identification information communication data, but transmits the pairing command.

The capsule receives the pairing command, checks whether the capsule characteristic identifier and the product code in the pairing command are consistent with the capsule characteristic identifier and the product code in local; when the capsule characteristic identifier and the product code in the pairing command are consistent with the capsule characteristic identifier and the product code in local, the product identification information communication data transmitted by the capsule is received by the corresponding configurator, and thus, pairing with that configurator can be performed; and, saves the configurator characteristic identifier in the pairing command, and adds the configurator characteristic identifier to the product identification information communication data and transmits the product identification information communication data.

The configurator receives the product identification information communication data and checks whether the configurator characteristic identifier in the product identification information communication data is consistent with the configurator characteristic identifier in local, and when the configurator characteristic identifier in the product identification information communication data is consistent with the configurator characteristic identifier in local, the configurator locks the capsule to complete pairing.

Further, in the step "the capsule receives the pairing command and checks whether the capsule characteristic identifier and the product code in the pairing command are consistent with the capsule characteristic identifier and product code in local", when the capsule characteristic identifier and the product code in the pairing command are consistent with the capsule characteristic identifier and the product code in local, the capsule is controlled to enter a data communication state, and the capsule periodically transmits the product identification information communication data and the capsule state communication data in turn, and both of the product identification information communication data and the capsule state communication data comprise the capsule state information.

After the step "the configurator locks the capsule" comprises:
the configurator saves the product identification information communication data and continuously acquires the capsule state information from the capsule state communication data.

Therefore, after the first verification, when the capsule characteristic identifier and the product code in the pairing command are consistent with the capsule characteristic identifier and the product code in local, the capsule needs to transmit the product identification information communication data so as to send pairing confirmation to the corresponding configurator. Also, the capsule state communication data is sent. The product identification information communication data and the capsule state communication data are periodically transmitted in turn at regular intervals (e.g., 2 seconds + N milliseconds). After the configurator and the capsule are interlocked, the configurator can continuously obtain the capsule state information from the capsule state communication data.

During time that the configurator and the capsule are interlocked, even if the configurator is powered off or restarted, etc., the configurator is not unlocked from the capsule. However, if the capsule is powered off, the configurator needs to be restarted and locked after re-establishing the connection according to the above steps.

Also, in accordance with the embodiments of the present invention, the capsule system comprises a user mobile terminal, and the user mobile terminal is provided with a capsule control APP (application), through which the user can control the configurator and make it connect with the capsule.

In the second embodiment of the present invention, the capsule can transmit a capsule broadcast packet and a capsule information packet under different circumstances, and the configurator can transmit a configurator pairing packet. The capsule broadcast packet is composed of 10 bytes, comprising 1 byte of the command characteristic identifier, 5 bytes of the capsule characteristic identifier bits, 2 bytes of a capsule power-on time and 2 bytes of the configurator characteristic identifier bits. The capsule information packet is composed of 13 bytes, comprising 1 byte of the command characteristic identifier, 5 bytes of the capsule characteristic identifier bits, 5 bytes of information data and 2 bytes of the configurator characteristic identifier bits. The configurator pairing packet is composed of 10 bytes, comprising 1 byte of the command characteristic identifier, 5 bytes of the capsule characteristic identifier bits, 2 bytes of a data communication channel and 2 bytes of the configurator characteristic identifier bits. The configurator characteristic identifier bits and the capsule characteristic identifier bits can be optionally filled in with the configurator characteristic identifier and the capsule characteristic identifier.

The command characteristic identifier is used to distinguish the type of data. The command characteristic identifier in the capsule broadcast packet is the product code identifier, the command characteristic identifier in the capsule information packet is the capsule state identifier and the command characteristic identifier in the configurator pairing packet is a pairing information identifier.

The information data in the capsule information packet comprises the operating state, the command confirmation, the test data, etc.

The capsule characteristic identifier is the product code.

Specifically, in the second embodiment, the communication method specifically comprises:
the capsule periodically transmits the capsule broadcast packet that comprises the capsule characteristic identifier; after power-on, the capsule is on a default wireless communication channel and is in an information broadcast mode, and therefore transmits the capsule broadcast packet periodically at fixed intervals (e.g., 3 seconds + N milliseconds) as a handshake signal, and at which time the configurator characteristic identifier bits in the capsule broadcast packet are empty.

The configurator can find the capsule by receiving the capsule broadcast packet, extract the capsule characteristic identifier and add it to the configurator pairing packet along with the configurator characteristic identifier and the data communication channel, and then transmit the configurator pairing packet. In this embodiment, the configurator may have at least two communication channels.

The capsule receives the configurator pairing packet, checks whether the capsule characteristic identifier in the configurator pairing packet is consistent with the capsule characteristic identifier in local, and when the capsule characteristic identifier in the configurator pairing packet is consistent with the capsule characteristic identifier in local, saves the configurator characteristic identifier locally and adds the configurator characteristic identifier to the capsule broadcast packet and transmits it, and transmits the capsule information packet through the data communication channel in the configurator pairing packet. The capsule broadcast packet can be used as a command to confirm the pairing, after which the wireless communication channel of the capsule is changed to the data communication channel and the capsule information packet is transmitted at fixed time intervals (e.g., 2 seconds + N milliseconds).

The configurator receives the capsule broadcast packet and checks whether the configurator characteristic identifier in the capsule broadcast packet is consistent with the configurator characteristic identifier in local, and when the configurator characteristic identifier in the capsule broadcast packet is consistent with the configurator characteristic identifier in local, the configurator locks the capsule to complete pairing.

In the second embodiment, the configurator comprises a first antenna and a second antenna, wherein the first antenna supports a monitoring channel and a data communication channel, and the second antenna supports the data communication channel. The monitoring channel is a fixed-frequency wireless channel, which is the default wireless communication channel that the capsule is on when first powered on, and is used to monitor the capsule broadcast packet. The data communication channel is a wireless channel with a frequency specified by the configurator after the configurator and the capsule are paired, and is dedicated to data interaction.

Therefore, before the step "the configurator locks the capsule" comprises:
the first antenna of the configurator is on the monitoring channel and the second antenna of the configurator is on the data communication channel.

After the step "the configurator locks the capsule" comprises:
the first antenna of the configurator and the second antenna of the configurator are both on the data communication channel, and receive the capsule information packet together to continuously obtain the capsule state information.

In addition, when there is interference in the wireless channel where the configurator is currently located, the configurator switches the wireless channel for communication with the capsule.

Therefore, in the second embodiment, the configurator comprises two antennas, and the two antennas are on different channels in different situations for different data communication.

During the time that the configurator and the capsule are interlocked, even if the configurator is powered off or restarted, it is not unlocked from the capsule. However, if the capsule is powered off, the configurator needs to be restarted and locked after re-establishing the connection according to the above steps.

Also, in accordance with the embodiments of the present invention, the capsule system comprises a user mobile terminal, and the user mobile terminal is provided with a capsule control application, through which the user can control the configurator and make it connect with the capsule.

Above, the method of interlocking and pairing the capsule and the configurator in the capsule system is described. Under certain circumstances, the capsule and the configurator can also be unpaired or change the pairing. The details can be described below.

After the step "the configurator locks the capsule" comprises: the capsule and the configurator are unpaired. Specifically, there are three embodiments for unpairing the capsule and the configurator.

In a first embodiment, the capsule control application in the user mobile terminal transmits the product code of the currently paired capsule to the configurator, the configurator transmits an unlock command to the currently paired capsule, the capsule receives the unlock command and transmits an unlock confirmation command to the configurator, the configurator receives the unlock confirmation command and unpairs with the capsule.

In a second embodiment, the configurator receives a mechanical key signal, the configurator transmits an unlock command to the currently paired capsule, the capsule receives the unlock command and transmits an unlock confirmation command to the configurator, the configurator receives the unlock confirmation command and unpairs with the capsule. Specifically, the user can press and hold a "STOP" button on the configurator for more than 3 seconds, and the configurator can receive the mechanical key signal that unpairing is needed.

In a third embodiment, the configurator monitors duration of the capsule power-on time, and the capsule is automatically unpaired with the configurator when the duration of the capsule power-on time reaches a threshold T. Specifically, when the duration of the capsule power-on time reaches 25 hours, the configurator is no longer needed, and the configurator transmits an unlock command to the currently paired capsule, then the capsule can be unpaired from the configurator automatically.

In the above three embodiments where the capsule is unpaired from the configurator, in the first and second embodiments, the unpaired capsule enters the information broadcast mode, and in the third embodiment, the unpaired capsule enters a cruise state and transmits the capsule information packet at fixed time intervals (e.g., 7 seconds + N milliseconds).

Alternatively, after the step "the configurator locks the capsule" comprises: the configurator changes the paired capsule.

Specifically, the step "the configurator changes the paired capsule" comprises:
a target configurator obtains the product code of a target capsule;
unpairing the current paired capsule of the target configurator;
when the target capsule is in an unpaired state, pairing the target configurator with the target capsule;
when the target capsule is in a paired state, the target configurator transmits a forced unpair command to the target capsule to unpair the target capsule with the current configurator, and then pairing the target configurator with the target capsule.

That is, in the case that the configurator and the capsule are paired with each other, it is still possible to change the paired capsule for the configurator.

And specifically, in the present invention, the step "a target configurator obtains the product code of a target capsule" comprises:
the user mobile terminal obtains the product code of the target capsule and transmits it to the configurator; or, the user mobile terminal receives the capsule searched by the target configurator and receives a selection information to select the target capsule, and then transmits the product code of the selected target capsule to the configurator. That is, the product code of the target capsule can be obtained by directly scanning the product code on the target capsule with user mobile terminal, or through search results of the user mobile terminal using the configurator, and after selected by the user, the product code of the target capsule is transmitted to the configurator.

To sum up, the present invention discloses a communication method for a capsule system. The capsule pairs the received capsule characteristic identifier with the capsule characteristic identifier in local, and when it is consistent, the capsule characteristic identifier transmitted by the capsule is received by a configurator, and the capsule and the configurator go to a first stage of verification; then, the configurator pairs the received configurator characteristic identifier with the configurator characteristic identifier in local, and when it is consistent, the configurator characteristic identifier transmitted by the configurator is received by the corresponding capsule, and the capsule and the configurator go to a second stage of verification. In addition, during the two verifications, the capsule saves the configurator characteristic identifier of the corresponding configurator, and the configurator saves the capsule characteristic identifier of the corresponding capsule. Therefore, the capsule and the configurator can be paired with each other, and subsequent communication between the two can be one-to-one without interference from other capsules or configurators.

Further, the present invention also provides two specific embodiments for pairing the capsule and the configurator. In addition, a method for unpairing and changing the pairing between the capsule and the configurator is provided, thereby making the communication method of the present invention more complete.

Furthermore, it should be understood that although the specification is described according to embodiments, not each embodiment contains only one separate technical solution, and that the specification is described in this manner only for clarity, and that those skilled in the art should consider the specification as a whole, and that the technical solutions in each embodiment may be suitably combined to form other embodiments as may be understood by those skilled in the art.

The series of detailed descriptions listed above are only specific to the feasible embodiments of the present invention and are not intended to limit the scope of protection of the present invention, and any equivalent embodiments or changes made without departing from the spirit of the art of the present invention shall be included in the scope of protection of the present invention.

## Claims

1. A communication method for a capsule system, comprising:
a capsule periodically transmits a capsule characteristic identifier;
a configurator receives the capsule characteristic identifier, and transmits the capsule characteristic identifier and a configurator characteristic identifier of the configurator;
the capsule receives the capsule characteristic identifier and checks whether the received capsule characteristic identifier is consistent with the capsule characteristic identifier in local, and the capsule saves the configurator characteristic identifier and transmits the configurator characteristic identifier when the received capsule characteristic identifier is consistent with the capsule characteristic identifier in local;
the configurator receives the configurator characteristic identifier and checks whether the received configurator characteristic identifier is consistent with the configurator characteristic identifier in local, and the configurator locks the capsule to complete pairing when the received configurator characteristic identifier is consistent with the configurator characteristic identifier in local.

2. The communication method of claim 1, wherein the communication method specifically comprises:
the capsule periodically transmits a capsule communication packet that comprises the capsule characteristic identifier and a product code;
the configurator receives the capsule communication packet, extracts the capsule characteristic identifier and the product code, adds them together with the configurator characteristic identifier to a pairing command, and transmits the pairing command;
the capsule receives the pairing command and checks whether the capsule characteristic identifier and the product code in the pairing command are consistent with the capsule characteristic identifier and the product code in local;
the capsule saves the configurator characteristic identifier in the pairing command and adds the configurator characteristic identifier to the capsule communication packet and transmits the capsule communication packet when the capsule characteristic identifier and the product code in the pairing command are consistent with the capsule characteristic identifier and the product code in local;
the configurator receives the capsule communication packet and checks whether the configurator characteristic identifier in the capsule communication packet is consistent with the configurator characteristic identifier in local, and the configurator locks the capsule to complete pairing when the configurator characteristic identifier in the capsule communication packet is consistent with the configurator characteristic identifier in local.

3. The communication method of claim 2, wherein the capsule communication packet comprises a command characteristic identifier, capsule characteristic identifier bits, capsule state information, and configurator characteristic identifier bits, with the capsule characteristic identifier bits optionally filled in with the capsule characteristic identifier and the configurator characteristic identifier bits optionally filled in with the configurator characteristic identifier; and wherein the capsule communication packet is product identification information communication data when the command characteristic identifier is a product code identifier; the capsule communication packet is capsule state communication data when the command characteristic identifier is a capsule state identifier.

4. The communication method of claim 3, wherein in the step "the capsule receives the pairing command and checks whether the capsule characteristic identifier and the product code in the pairing command are consistent with the capsule characteristic identifier and the product code in local", the capsule is controlled to enter a data communication state when the capsule characteristic identifier and the product code in the pairing command are consistent with the capsule characteristic identifier and the product code in local, and the capsule periodically transmits the product identification information communication data and the capsule state communication data in turn, and both of the product identification information communication data and the capsule state communication data comprise the capsule state information;
after the step "the configurator locks the capsule" comprises:
the configurator saves the product identification information communication data and continuously acquires the capsule state information from the capsule state communication data.

5. The communication method of claim 1, wherein the communication method specifically comprises:
the capsule periodically transmits a capsule broadcast packet that comprises the capsule characteristic identifier;
the configurator finds the capsule, extracts the capsule characteristic identifier and adds it to a configurator pairing packet along with the configurator characteristic identifier and a data communication channel, and transmits the configurator pairing packet;
the capsule receives the configurator pairing packet and checks whether the capsule characteristic identifier in the configurator pairing packet is consistent with the capsule characteristic identifier in local,
and the capsule saves the configurator characteristic identifier locally and adds the configurator characteristic identifier to the capsule broadcast packet and transmits it when the capsule characteristic identifier in the configurator pairing packet is consistent with the capsule characteristic identifier in local, and transmits a capsule information packet through the data communication channel in the configurator pairing packet;
the configurator receives the capsule broadcast packet and checks whether the configurator characteristic identifier in the capsule broadcast packet is consistent with the configurator characteristic identifier in local, and the configurator locks the capsule to complete pairing when the configurator characteristic identifier in the capsule broadcast packet is consistent with the configurator characteristic identifier in local.

6. The communication method of claim 5, wherein before the step "the configurator locks the capsule to complete the pairing" comprises:
a first antenna of the configurator is on a monitoring channel and a second antenna of the configurator is on the data communication channel;
after the step 'the configurator locks the capsule" comprises:
the first antenna of the configurator and the second antenna of the configurator are both on the data communication channel, and receive the capsule information packet together to continuously obtain capsule state information.

7. The communication method of claim 5, wherein the capsule broadcast packet comprises a command characteristic identifier, capsule characteristic identifier bits, a capsule power-on time, and configurator characteristic identifier bits; the configurator pairing packet comprises the command characteristic identifier, the capsule characteristic identifier bits, the data communication channel, and the configurator characteristic identifier bits; the capsule information packet comprises the command characteristic identifier, the capsule characteristic identifier bits, information data, and the configurator characteristic identifier bits; the capsule characteristic identifier bits are optionally filled in with the capsule characteristic identifier and the configurator characteristic identifier bits are optionally filled in with the configurator characteristic identifier.

8. The communication method of claim 1, wherein after the step "the configurator locks the capsule" comprises:
a user mobile terminal transmits a product code of the currently paired capsule to the configurator, the configurator transmits an unlock command to the currently paired capsule, the capsule receives the unlock command and transmits an unlock confirmation command to the configurator, the configurator receives the unlock confirmation command and unpairs with the capsule;
or, the configurator receives a mechanical key signal, the configurator transmits an unlock command to the currently paired capsule, the capsule receives the unlock command and transmits an unlock confirmation command to the configurator, the configurator receives the unlock confirmation command and unpairs with the capsule;
or, the configurator monitors duration of capsule power-on time, and the capsule is automatically unpaired with the configurator when the duration of the capsule power-on time reaches a threshold T.

9. The communication method of claim 1, wherein after the step "the configurator locks the capsule" comprises:
a target configurator obtains a product code of a target capsule;
unpairing the current paired capsule of the target configurator;
pairing the target configurator with the target capsule when the target capsule is in an unpaired state;
the target configurator transmits a forced unpair command to the target capsule to unpair the target capsule with the current configurator when the target capsule is in a paired state, and then pairing the target configurator with the target capsule.

10. The communication method of claim 9, wherein the step "a target configurator obtains a product code of a target configuration capsule" comprises: the user mobile terminal obtains the product code of the target capsule and transmits it to the configurator; or, the user mobile terminal receives the capsule searched by the target configurator and receives a selection information to select the target configuration capsule, and then transmits the product code of the selected target configuration capsule to the configurator.
